# FASCICULE DE BREVET EUROPEEN

(11) **EP 1 095 020 B1**
(45) Date de publication et mention de la délivrance du brevet: **31.08.2005**
(21) Numéro de dépôt: 99929395.4
(22) Date de dépôt: 05.07.1999
(51) Int. Cl.: C07D 213/73, C07D 405/12, A61K 31/44, A61K 31/495, C07D 401/04, C07C 233/44, C07C 271/22

(54) **DERIVES DE 2-AMINOPYRIDINES, LEUR UTILISATION EN TANT QUE MEDICAMENTS ET COMPOSITIONS PHARMACEUTIQUES LES CONTENANT**
4-AMINOPYRIDINDERIVATE, IHRE VERWENDUNG ALS ARZNEIMITTEL UND DIESE ENTHALTENDE PHARMAZEUTISCHE ZUSAMMENSETZUNGEN
2-AMINOPYRIDINE ERIVATIVES, THEIR USE AS MEDICINES AND PHARMACEUTICAL COMPOSITIONS CONTAINING THEM

(30) Priorité: 08.07.1998 FR 9808732; 02.04.1999 FR 9904133
(43) Date de publication de la demande: 02.05.2001
(73) Titulaire: SOCIETE DE CONSEILS DE RECHERCHES ET D'APPLICATIONS SCIENTIFIQUES (S.C.R.A.S.), 75016 Paris (FR)
(72) Inventeur: CHABRIER DE LASSAUNIERE, Pierre-Etienne, F-75016 Paris (FR); AUVIN, Serge, F-91370 Mauchamps (FR); HARNETT, Jerry, F-91190 Gif sur Yvette (FR); PONS, Dominique, F-75014 Paris (FR); ULIBARRI, Gérard, F-91440 Bures-sur-Yvette (FR); BIGG, Dennis, F-91190 Gif-sur-Yvette (FR)
(74) Mandataire: Bourgouin, André
(86) Numéro de dépôt international: PCT/FR1999/001610
(87) Numéro de publication internationale: WO 2000/002860

(56) Documents cités:
- EP-A- 0 790 240
- WO-A-96/18616
- WO-A-96/30350
- WO-A-98/24766
- US-A- 3 037 988

## Description

La présente invention a pour objet des nouveaux dérivés de 2-aminopyridines présentant une activité inhibitrice des enzymes NO-synthases produisant le monoxyde d'azote NO et / ou une activité piégeuse des formes réactives de l'oxygène (ROS pour "*reactive oxygen species*"). L'invention concerne les dérivés correspondant à la formule générale **(I)** définie plus loin, leurs méthodes de préparation, les préparations pharmaceutiques les contenant et leur utilisation à des fins thérapeutiques, en particulier leur utilisation en tant qu'inhibiteurs des NO-synthases et piégeurs de formes réactives de l'oxygène de manière sélective ou non.

Compte tenu du rôle potentiel du NO et des ROS en physiopathologie, les nouveaux dérivés décrits répondant à la formule générale **(I)** peuvent produire des effets bénéfiques ou favorables dans le traitement de pathologies où ces espèces chimiques sont impliquées. Notamment :
- dans le traitement de troubles cardio-vasculaires et cérébro-vasculaires comprenant par exemple l'athérosclérose, la migraine, l'hypertension artérielle, le choc septique, les infarctus cardiaques ou cérébraux d'origine ischémique ou hémorragiques, les ischémies et les thromboses ;
- dans le traitement de troubles du système nerveux central ou périphérique comme par exemple les maladies neurodégénératives où l'on peut notamment citer les infarctus cérébraux, l'hémorragie sub arachnoïde, le vieillissement, les démences séniles, y compris la maladie d'Alzheimer, la chorée de Huntington, la maladie de Parkinson, la maladie de Creutzfeld Jacob et les maladies à prions, la sclérose latérale amyotrophique mais aussi la douleur, les traumatismes cérébraux ou de la moelle épinière, l'addiction aux opiacées, à l'alcool et aux substances induisant une accoutumance, les troubles de l'érection et de la reproduction, les désordres cognitifs, les encéphalopathies, les encéphalopathies d'origine virale ou toxique
- dans le traitement de troubles du muscle squelettique et des jonctions neuromusculaires (myopathie, myosite) ainsi que les maladies cutanées ;
- dans le traitement de maladies prolifératives et inflammatoires comme par exemple l'athérosclérose, l'hypertension pulmonaire, la détresse respiratoire la glomérulonéphrite, l'hypertension portale, le psoriasis, l'arthrose et l'arthrite rhumatoïde, les fibroses, les amyloïdoses, les inflammations du système gastro-intestinal (colite, maladie de Crohn) ou du système pulmonaire et des voies aériennes (asthme, sinusites, rhinites) ;
- dans les traitements en relation avec des transplantations d'organes ;
- dans le traitement de maladies auto-immunes et virales comme par exemple le lupus, le SIDA, les infections parasitaires et virales, le diabète, la sclérose en plaques ;
- dans le traitement du cancer ;
- dans le traitement de maladies neurologiques associées à des intoxications (empoisonnement au Cadmium, inhalation de n-hexane, pesticide, herbicide), à des traitements (radiothérapie) ou à des désordres d'origine génétique (maladie de Wilson) ;
- dans le traitement de toutes les pathologies caractérisées par une production excessive ou un dysfonctionnement de NO et/ou des ROS.

Dans l'ensemble de ces pathologies, il existe des évidences expérimentales démontrant l'implication du NO ou des ROS (*J. Med. Chem.* (1995) **38**, 4343-4362 ; *Free Radic. Biol. Med.* (1996) **20**, 675-705 ; *The Neuroscientist* (1997) 3, 327-333).

Par ailleurs les inventeurs ont déjà décrit dans des brevets antérieurs des inhibiteurs de NO Synthases et leur utilisation (US patent 5,081,148; US patent 5,360,925), ainsi que l'association de ces inhibiteurs avec des produits possédant des propriétés antioxydantes ou antiradicalaires (demande de brevet WO 98 / 09653). Plus récemment, ils ont décrit dans les demandes de brevet WO 98/42696 et WO 98/58934 des dérivés d'amidines possédant des propriétés d'inhibition de NO Synthases et / ou des propriétés antioxydantes ou antiradicalaires.

La demanderesse a maintenant découvert une nouvelle classe de composés présentant une activité inhibitrice des NO-synthases et / ou une activité piégeuse des formes réactives de l'oxygène (ROS pour "*reactive oxygen species* "). Ces composés sont des dérivés de 2-aminopyridines.

Des 2-amino-pyridines de structure similaire à celle des composés selon l'invention, sont décrits dans la littérature : par exemple le brevet US 3,037,988 décrit des agents hypotenseurs ; les demandes WO96/18616, WO 98/24766 et WO 96/30350 décrivent des inhibiteurs des NO synthases et EP 790240 des piégeurs des formes réactives de l'oxygène. Les composés possèdent l'activité duale d'inhiber les NOS et de piéger les ROS.

La présente invention a pour objet un composé choisi par les composés suivants :
- 6-amino-N-[3,5-bis-(1,1-diméthyléthyl)-4-hydroxyphényl]-4-méthyl-2-pyridinepentanamide ;
- N-[(6-amino-4-méthyl-2-pyridinyl)butyl]-2,5-dihydroxy-3-(1-méthyléthyl)-benzamide ;
- 6-amino-N-[4-(diméthylamino)phényl]-4-méthyl-2-pyridinehexanamide ;
- 6-amino-N-[4-(diméthylamino)phényl]-4-méthyl-2-pyridinepentananamide ;
- 6-amino-N-[3-(4-hydroxy-3-méthoxy-phényl)-2-propényl]-4-méthyl-2-pyridinebutanamine ;
- 2-({[4-(6-amino-4-méthyl-2-pyridinyl)butyl]amino}méthyl)-4,5-diméthoxyphénol ;
ou un sel d'addition d'un de ces composés à des acides organiques ou inorganiques ou à des bases.

Dans certains cas, les composés selon la présente invention peuvent comporter des atomes de carbone asymétriques, et donc posséder deux formes énantiomères possibles, c'est-à-dire les configurations "R" et "S". La présente invention inclut les deux formes énantiomères et toutes combinaisons de ces formes, y compris les mélanges racémiques "RS". Dans un souci de simplicité, lorsqu'aucune configuration spécifique n'est indiquée dans les formules de structure, il faut comprendre que les deux formes énantiomères et leurs mélanges sont représentés.

L'invention a également pour objet, à titre de médicaments, les composés tels que décrits précédemment ou leurs sels pharmaceutiquement acceptables. Elle concerne aussi des compositions pharmaceutiques contenant, à titre de principe actif, ces composés ou leurs sels pharmaceutiquement acceptables, et l'utilisation de ces composés ou de leurs sels pharmaceutiquement acceptables pour fabriquer des médicaments destinés à inhiber la NO synthase, à inhiber la péroxidation lipidique ou ayant à la fois une activité d'inhibition de la NO synthase et d'inhibition de la péroxidation lipidique.

L'invention a également pour objet l'utilisation d'un composé tel que défini ci-dessus, ou d'un sel pharmaceutiquement acceptable d'un tel composé, pour fabriquer un médicament destiné à traiter une pathologie choisie parmi les troubles cardio-vasculaires et cérébro-vasculaires, les troubles du système nerveux central ou périphérique, les troubles du muscle squelettique et des jonctions neuromusculaires, les maladies cutanées, les maladies prolifératives et inflammatoires, les maladies auto-immunes et virales et les maladies neurologiques associées à des intoxications, à des traitements ou à des désordres d'origine génétique.

De préférence, l'invention a pour objet l'utilisation telle définie ci-dessus, caractérisée en ce que le médicament fabriqué est destiné à traiter des troubles cardio-vasculaires et cérébro-vasculaires, et de manière très préférentielle, les troubles cardio-vasculaires et cérébro-vasculaires sont choisis parmi l'athérosclérose, la migraine, l'hypertension artérielle, le choc septique, les infarctus cardiaques ou cérébraux d'origine ischémique ou hémorragiques, les ischémies et les thromboses.

De préférence également, l'invention a pour objet l'utilisation telle définie ci-dessus, caractérisée en ce que le médicament fabriqué est destiné à traiter des troubles du système nerveux central ou périphérique, et de manière très préférentielle, les troubles du système nerveux central ou périphérique sont choisis parmi les infarctus cérébraux, l'hémorragie sub arachnoïde, le vieillissement, les démences séniles, la maladie d'Alzheimer, la chorée de Huntington, la maladie de Parkinson, la maladie de Creutzfeld Jacob, les maladies à prions, la sclérose latérale amyotrophique, la douleur, les traumatismes cérébraux ou de la moelle épinière, l'addiction aux opiacées, à l'alcool et aux substances induisant une accoutumance, les troubles de l'érection et de la reproduction, les désordres cognitifs et les encéphalopathies.

De préférence également, l'invention a pour objet l'utilisation telle définie ci-dessus, caractérisée en ce que le médicament fabriqué est destiné à traiter la sclérose en plaques.

Par sel pharmaceutiquement acceptable, on entend notamment des sels d'addition d'acides inorganiques tels que chlorhydrate, sulfate, phosphate, diphosphate, bromhydrate et nitrate ou d'acides organiques tels que acétate, maléate, fumarate, tartrate, succinate, citrate, lactate, méthane sulfonate, p-toluènesulfonate, pamoate, oxalate et stéarate. Entrent également dans le champ de la présente invention, lorsqu'ils sont utilisables, les sels formés à partir de bases telles que l'hydroxyde de sodium ou de potassium. Pour d'autres exemples de sels pharmaceutiquement acceptables, on peut se référer à "Pharmaceutical salts", *J Pharm. Sci*. **66**:1 (1977).

La composition pharmaceutique peut être sous forme d'un solide, par exemple des poudres, des granules, des comprimés, des gélules, des liposomes ou des suppositoires. Les supports solides appropriés peuvent être, par exemple, le phosphate de calcium, le stéarate de magnésium, le talc, les sucres, le lactose, la dextrine, l'amidon, la gélatine, la cellulose, la cellulose de méthyle, la cellulose carboxyméthyle de sodium, la polyvinylpyrrolidine et la cire.

Les compositions pharmaceutiques contenant un composé de l'invention peuvent aussi se présenter sous forme liquide, par exemple, des solutions, des émulsions, des suspensions ou des sirops. Les supports liquides appropriés peuvent être, par exemple, l'eau, les solvants organiques tels que le glycérol ou les glycols, de même que leurs mélanges, dans des proportions variées, dans l'eau.

L'administration d'un médicament selon l'invention pourra se faire par voie topique, orale, parentérale, par injection intramusculaire, etc.

La dose d'administration envisagée pour médicament selon l'invention est comprise entre 0,1 mg à 10 g suivant le type de composé actif utilisé.

Conformément à l'invention, on peut préparer les composés de formule générale **(I)** par le procédé décrit ci-dessous.

### Préparation des composés de formule générale (I) :

Les composés selon l'invention (de formule générale **(I)** dans le schéma ci-dessous), dans lesquels
A représente un radical 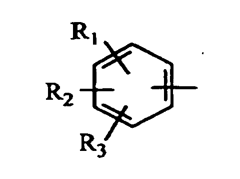 dans lequel R₁, R₂ et R₃ représentent, indépendamment, le groupe OH, un radical alkyle ou alkoxy linéaire ou ramifié ayant de 1 à 6 atomes de carbone, ou -NR₅R₆ ;
R₅ et R₆ représentant, indépendamment, un radical alkyle linéaire ou ramifié ayant de 1 à 6 atomes de carbone ;
X représente un radical -(CH₂)ₘ-Q-, -(CH₂)ₘ-CH=CH-Q-, -(CH₂)ₘ-C(=W)-Q-, -(CH₂)ₘ-NR₁₁-C(=W)-Q- ;
Y représente une chaîne alkyle linéaire ou ramifiée contenant jusqu'à 10 atomes de carbone, ou -(CH₂)ₙ-NR₁₃-(CH₂)ₚ-,
m, n et p étant des entiers compris entre 0 et 6 ;
Q représentant une liaison ;
W représentant l'atome d'oxygène ;
R₁₀ représente un radical alkyle linéaire ou ramifié ayant de 1 à 6 atomes de carbone ;
R₁₁ et R₁₃ représentent un atome d'hydrogène ;
et les radicaux A, X, Y et R₁₀ sont tels que la formule I représente l'un des 6 produits suivants :
- 6-amino-N-[3,5-bis-(1,1-diméthyléthyl)-4-hydroxyphényl]-4-méthyl-2-pyridinepentanamide ;
- N-[(6-amino-4-méthyl-2-pyridinyl)butyl]-2,5-dihydroxy-3-(1-méthyléthyl)-benzamide ;
- 6-amino-N-[4-(diméthylamino)phényl]-4-méthyl-2 pyridinehexanamide ;
- 6-amino-N-[4-(diméthylamino)phényl]-4-méthyl-2-pyridinepentananamide ;
- 6-amino-N-[3-(4-hydroxy-3-méthoxy-phényl)-2-propényl]-4-méthyl-2-pyridinebutanamine ;
- 2-({[4-(6-amino-4-méthyl-2-pyridinyl)butyl]amino}méthyl)-4,5-diméthoxyphénol ;
peuvent être préparés à partir des intermédiaires de formule générale **(II)** ou des intermédiaires de formule générale **(III)** et **(IV)** selon le schéma 1.

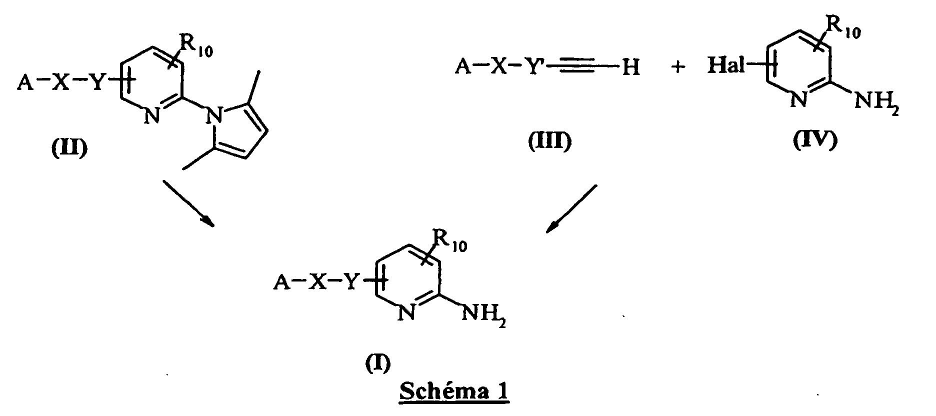

Par alkyle linéaire ou ramifié ayant de 1 à 6 atomes de carbone, on entend en particulier les radicaux méthyle, éthyle, propyle, isopropyle, butyle, isobutyle, sec-butyle et tert-butyle, pentyle, néopentyle, isopentyle, hexyle, isohexyle. Par radical alkoxy linéaire ou ramifié ayant de 1 à 6 atomes de carbone, on entend les radicaux dont le radical alkyle a la signification indiquée précédemment.

Les molécules finales de formule générale **(I)** sont obtenues après coupure du groupe protecteur 2,5-diméthyl pyrrole des composés de formule générale **(II)** par chauffage en présence de chlorhydrate d'hydroxylamine, à une température variant de 60° C à 100° C, dans un solvant tel que par exemple l'éthanol selon un protocole expérimental décrit dans *J. Chem. Soc. Perkin Trans.* (1984), 2801-2807. Lorsque les composés de formule générale **(I)** portent une amine protégée par un groupe labile en milieu acide (par exemple : carbamate de *tert*-butyle), celle-ci est libérée lors de l'étape finale de salification effectuée, dans ce cas, à l'aide d'un acide fort, en particulier HCl.

Alternativement les composés de formule générale **(I)** peuvent être obtenus par chauffage des alcynes de formule générale **(III)** avec les intermédiaires halogèno-pyridines de formule générale **(IV),** soit en présence de dérivés du Palladium (0), tel que Pd(PPh3)4 en opérant sous atmosphère inerte dans la n-butylamine, soit en présence de dérivé du Palladium **(II)**, tel que Pd(OAc)₂, et de PPh₃ dans la pipéridine (*J. Med. Chem.,* (1996), **36** (16), 3179-3187). Les dérivés acétyléniques de formule générale **(I)** ainsi obtenus peuvent éventuellement être transformés en dérivés éthyléniques par réduction soit sous atmosphère d'hydrogène en présence d'un catalyseur de type Lindlar soit par réduction en présence d'un hydrure tel que le RedAl (*J. Org. Chem.,* (1988), **53**, 3845). Les composés acétyléniques de formule générale **(I)** peuvent également être réduits par le Pd/C sous atmosphère d'hydrogène dans un solvant alcoolique tel que l'éthanol pour conduire directement aux alcanes correspondants.

Lorsque les composés de formule générale **(III)** portent une amine protégée (principalement sous forme de carbamate de *tert*-butyle), celle-ci est libérée après la condensation **(III)** + **(IV)** lors de l'étape finale de salification des molécules en présence d'un acide fort (par exemple HCl).

### Préparation des composés de formule générale (II), (III) et (IV) :

### A) Les composés de formule générale (II) peuvent être préparés selon les méthodes suivantes :

Les précurseurs synthétiques qui conduisent aux intermédiaires de formule générale **(II)** sont préparés à partir des composés de formule générale **(II.1),** tel que par exemple la 2-(2,5-diméthylpyrrol-1-yl)-4,6-diméthylpyridine. Celle-ci est obtenue à partir de la 6-amino-2,4-lutidine commerciale selon un protocole expérimental décrit dans *J. Chem. Soc. Perkin Trans*., (1984), **12**, 2801-2807. Le traitement des composés de formule générale **(II.1)** par une base forte telle que, par exemple, nBuLi, à une température variant de -50° C à -30° C dans un solvant anhydre tel que l'éther éthylique, sous atmosphère inerte et en présence éventuellement de N,N,N',N'-tétraméthyléthylènediamine permet de former le dérivé lithié (intermédiaire **(II.2)**) qui en présence d'un électrophile E⁺ conduit aux adduits de formule générale **(II.X).**

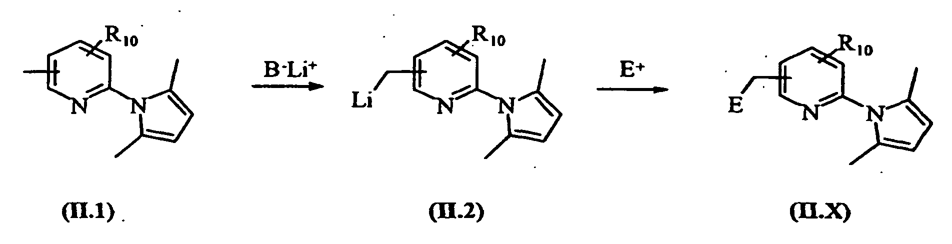

Parmi les électrophiles E⁺ qui peuvent réagir sur l'espèce lithiée de formule générale **(II.2),** on peut citer par exemple le CO₂, les halogéno-esters, les halogéno-orthoesters, le paraformaldéhyde, les halogéno-alcools protégés (par exemple sous forme d'acétal de tétrahydropyranne) ou les halogéno-amines protégées..

### 1) Méthodes d'accès aux 2-(2,5-diméthylpyrrol-1-yl)pyridines substituées de formule générale (II.X) :

### 1.1) Préparation des alcools de formule générale (II.3) :

L'action du dérivé **(II.2)** sur le paraformaldéhyde ou sur les halogèno-alcools protégés permet d'accéder, après éventuelle déprotection, aux alcools de formule générale **(II.3)**, dans lesquels Y et R₁₀ sont tels que définis ci-dessus.

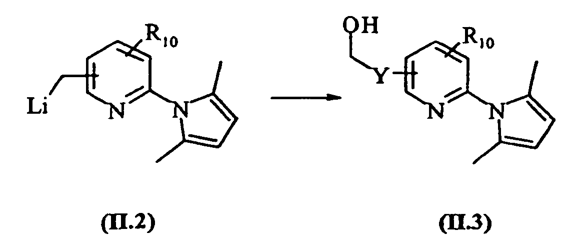

### 1.2) Préparation des aldéhydes de formule générale (II.4) :

Les aldéhydes de formule générale **(II.4),** dans lesquels Y et R₁₀ sont tels que définis ci-dessus, peuvent être préparés par oxydation des alcools de formule générale **(II.3)** et en utilisant, par exemple le chlorure d'oxalyle dans le DMSO (oxydation de Swern) ou bien un complexe pyridine-sulfurtrioxyde en présence d'une base telle que la triéthylamine (*Tetrahedron Lett.,* (1982), **23**, 807) :

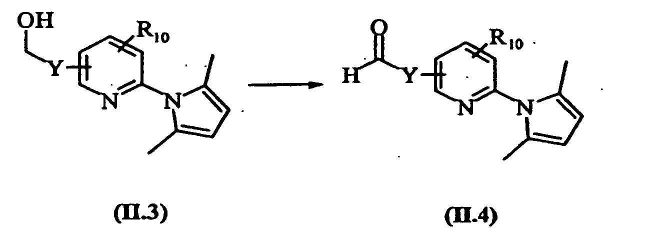

Ou bien ces aldéhydes sont également accessibles par la condensation des intermédiaires de formule générale **(II.2)** avec des dérivés de type halogéno-acétals suivie par une étape de déprotection classique en milieu acide :

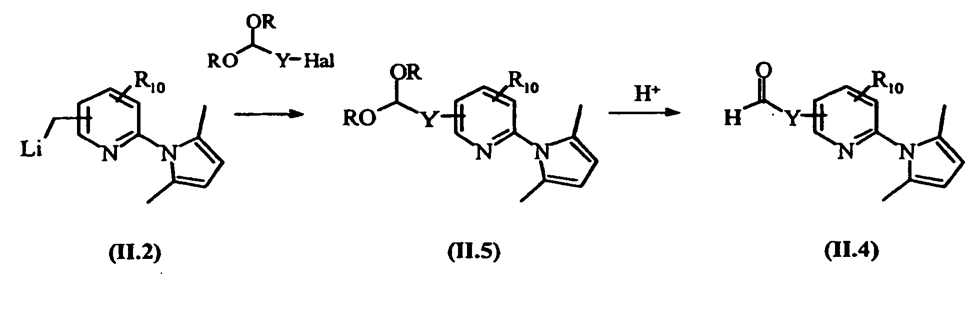

### 1.3) Préparation des acides carboxyliques de formule générale (II.6) :

L'action de l'intermédiaire **(II.2)** sur le CO₂ et sur les dérivés halogéno-esters ou orthoesters permet d'accéder, après éventuelle déprotection, aux acides carboxyliques de formule générale **(II.6)**, dans lesquels Y et R₁₀ sont tels que définis ci-dessus :

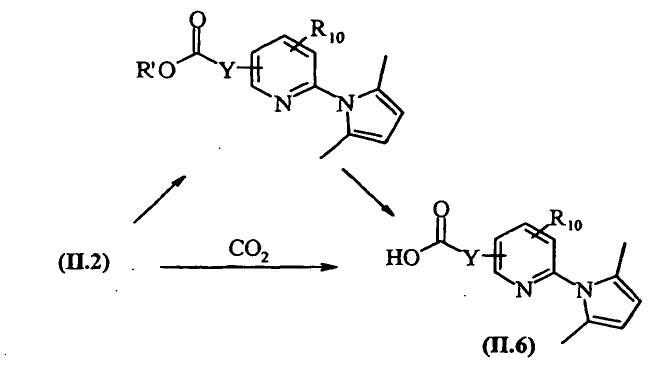

Ces acides peuvent également être obtenus par l'oxydation des aldéhydes de formule générale **(II.4)** par le nitrate d'argent selon un protocole expérimental décrit dans *J. Org. Chem.,* (1985), **50**, 2981-2987.

### 1.4) Préparation des amines de formule générale (II.7) :

Les alcools de formule générale **(II.3)**, précédemment décrits, permettent d'accéder aux amines de formule générale **(II.7)** dans lesquels Q, Y et R₁₀ sont tels que définis ci-dessus. La fonction alcool est classiquement activée sous forme d'un dérivé sulfonate de formule générale **(II.8)** avant d'être déplacée par une amine et en particulier une amine hétérocyclique. La condensation s'effectue en présence de carbonate de Césium et de LiI à une température de 70 °C à 100 °C et en particulier à reflux de la butanone. Les hétérocycles tels que la pipérazine sont utilisés sous forme mono-protégés (par ex. Boc) lors de la condensation et une étape supplémentaire de déprotection sélective est alors nécessaire pour libérer la deuxième fonction amine :

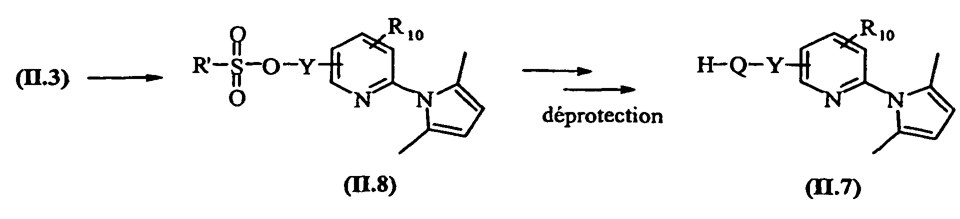

Dans le cas particulier où Q est une simple liaison et Y = -HN(R₁₃)-(CH₂)ₚ-, les amines de formule générale **(II.7)** sont préparées à partir de l'intermédiaire **(II.2)** qui est condensé sur des halogéno-amines protégées (par exemple sous forme de dérivés silylés ou phtalimides) dans des conditions précédemment décrites. Les amines primaires de formule générale **(II.7)** sont finalement obtenues après déprotection dans des conditions décrites dans la littérature (T.W. Greene et P.G.M. Wuts, Protective Groups in Organic Synthesis, Second edition (Wiley-Interscience, 1991)).

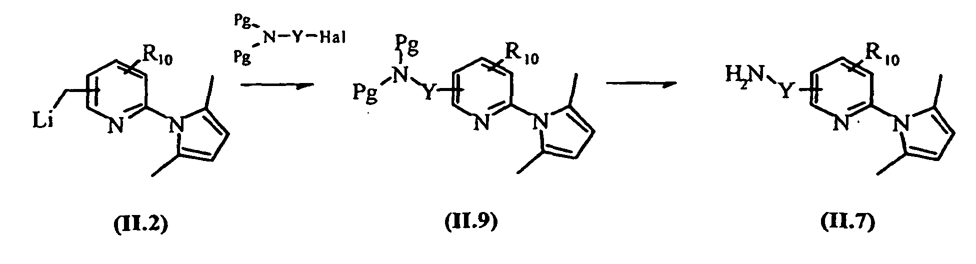

### 2) Méthodes d'accès aux composés de formule générale (II) :

### 2.1) Carboxamides de formule générale (II) :

**2.1.1)** Les carboxamides de formule générale **(II)**, dans lesquels A, X, Y et R₁₀ sont tels que définis ci-dessus, sont préparés par condensation des amines de formule générale **(A.1)** avec les acides de formule générale **(II.6)**, précédemment décrits, selon les méthodes classiques utilisées en condensation peptidique (M. Bodanszky et A. Bodanszky, The Practice of Peptide Synthesis (Springer-Verlag, 1984)). La synthèse des amines de formule générale **(A.1),** non commerciales, est décrite plus loin. 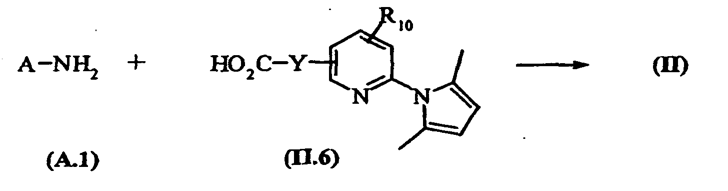
**2.1.2)** Les carboxamides de formule générale **(II),** dans lesquels A, X, Y et R₁₀ sont tels que définis ci-dessus, peuvent également être préparés par condensation des acides carboxyliques de formule générale **(A.2)** avec les amines de formule générale **(II.7)** dans les conditions précédemment décrites. La synthèse des acides carboxyliques de formule générale **(A.2)**, non commerciaux, est décrite plus loin.

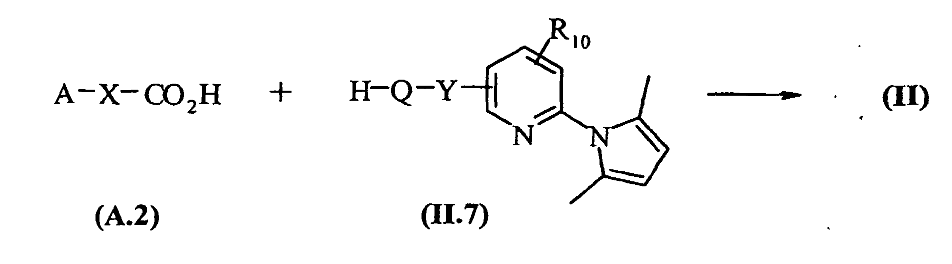

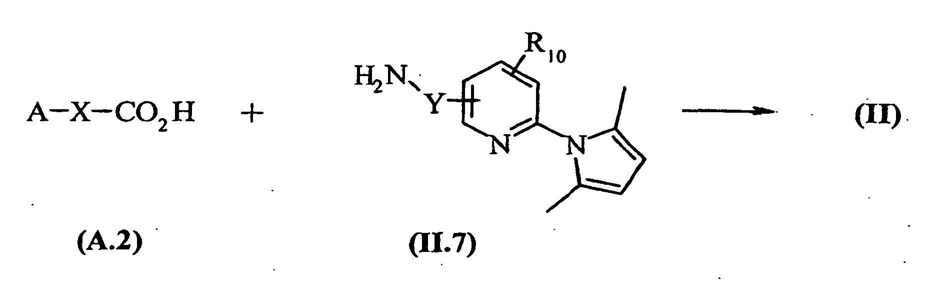

### 2.2) Amines de formule générale (II) :

**2.2.1)** Les amines de formule générale **(II),** dans lesquels A, X, Y et R₁₀ sont tels que définis ci-dessus, sont préparées par condensation d'une amine de formule générale **(A.1)** ou **(A.3)** avec un aldéhyde de formule générale **(II.4)** au cours d'une étape d'amination réductrice en présence d'un réducteur, tel que par exemple le borohydrure de sodium ou le triacétoxyborohydrure de sodium et dans un solvant tel que, par exemple, le 1,2-dichloroéthane. La synthèse des amines de formule générale **(A.3)** est décrite plus loin. 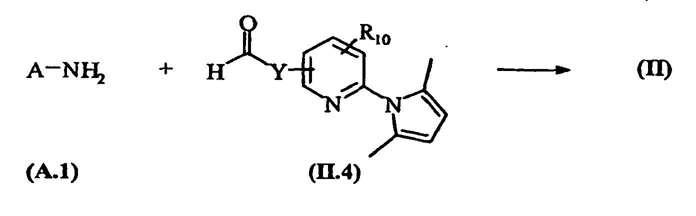 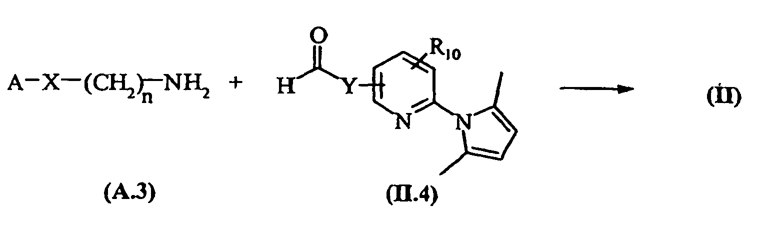
**2.2.2)** Les amines de formule générale **(II)**, dans lesquels A, X, Y et R₁₀ sont tels que définis ci-dessus, peuvent également être préparées par condensation des aldéhydes de formule générale **(A.4)** ou des dérivés de cinnamaldéhydes de formule générale **(A.5)** avec les amines de formule générale **(II.7)** dans les conditions précédemment décrites :

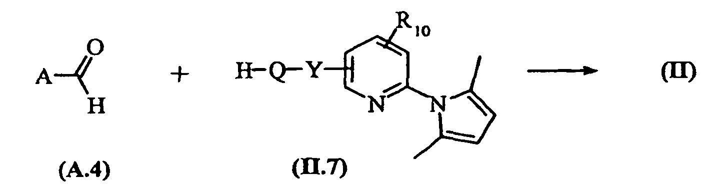

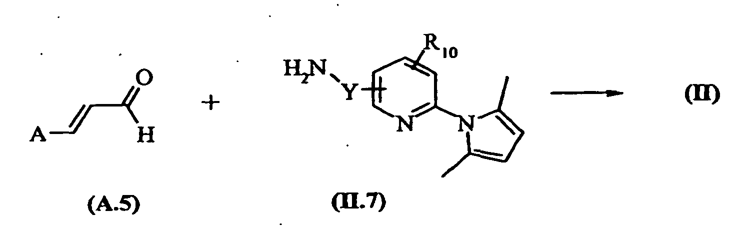

### 3) Métliodes d'accès aux intermédiaires de formule générale (A.X):

### 3.1) Synthèse des amines de formule générale (A.1) :

Dans le cas particulier où A est un dérivé phénolique, les anilines de formule générale **(A.1)** sont obtenues par hydrogénation, en présence d'une quantité catalytique de Pd/C, des dérivés nitrophénols correspondants, eux-mêmes synthétisés selon une méthode décrite dans la littérature (*J. Org. Chem*., (1968), **33** (1), 223-226).

Les amino-diphénylamines de formule générale **(A.1)** sont accessibles à partir des méthodes décrites dans la littérature *(Synthesis* (1990) 430 ; *Indian J. Chem*. (1981) **20B,** 611-613 ; *J. Med. Chem.* (1975) **18(4),** 386-391). Les nitro-diphénylamines intermédiairement obtenus conduisent, soit par hydrogénation catalytique, soit à l'aide de SnCl₂ (*J. Heterocyclic Chem.* (1987), **24,** 927-930; *Tetrahedron Letters* (1984), 25, (8), 839-842) aux amino-diphénylamines de formule générale **(A.1)**.

### 3.2) Synthèse des acides carboxyliques de formule générale (A.2) :

Les acides carboxyliques de formule générale **(A.2)** peuvent être préparés selon des méthodes décrites dans la littérature : *Can. J. Chem*. (1972), **50**, 1276-1282, *J. Org. Chem.* (1961) **26**, 1221-1223 ou *Acta Chem. Scandinavica* (1973) **27,** 888-890.

### 3.3) Synthèse des amines de formule générale (A.3) :

Les amines de formule générale **(A.3),** dans lesquelles A, X et n sont tels que décrits précédemment, sont préparés, en deux étapes, par condensation des amines de formule générale **(A.1)** avec les aminoacides protégés, commerciaux, de formule générale **(A.6)** dans les conditions classiques de la synthèse peptidique précédemment décrite. La déprotection de l'amine terminale de l'intermédiaire de formule générale **(A.7)** est alors effectuée lors de la dernière étape, et par exemple en milieu acide fort pour couper la fonction *tert*-butoxycarbonyle :

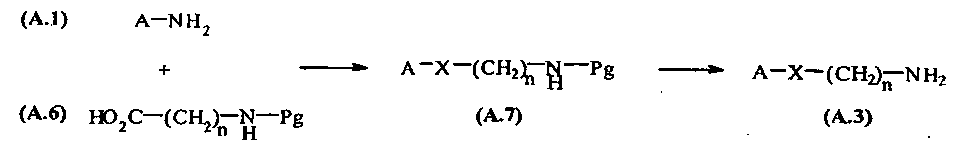

### B) Les composés de formule générale (III) peuvent être préparés selon les méthodes suivantes :

Les composés acétyléniques de formule générale **(III),** dans lesquels A, Q, W, Y et m sont tels que définis ci-dessus, sont préparés par substitution nucléophile des dérivés acétyléniques commerciaux de formule générale **(B.1),** dans lesquels Gp est un groupe labile tel que halogène ou dérivés sulfoniques, par une amine de formule générale **(B.2)** selon une procédure décrite dans *J. Med. Chem.,* (1996), **39** (16), 3179-3187.

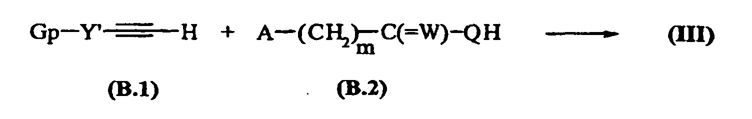

Les amines de formule générale **(B.2)** sont aisément accessibles à partir de méthodes décrites dans la littérature (p. ex. : *J. Med Chem.,* (1992), **35** (23), 4464-4472).

### C) Les composés de formule générale (IV) peuvent être préparés selon les méthodes suivantes :

Les dérivés halogénés des 2-aminopyridines de formule générale **(IV)**, dans lesquels R₁₀ est tel que défini ci-dessus, non commerciaux, peuvent être préparés selon des méthodes de la littérature et en particulier celles décrites dans *J. Org. Chem.,* (1962), **27**, 2473-2478, *Rec. Trav. Chim.,* (1966), **85,** 803 ou *Aust. J. Chem.,* (1982), **25**, 2025-2034.

A moins qu'ils ne soient définis d'une autre manière, tous les termes techniques et scientifiques utilisés ici ont la même signification que celle couramment comprise par un spécialiste ordinaire du domaine auquel appartient cette invention. De même, toutes les publications, demandes de brevets, tous les brevets et toutes autres références mentionnées ici sont incorporées par référence.

Les exemples suivants sont présentés pour illustrer les procédures ci-dessus et ne doivent en aucun cas être considérés comme une limite à la portée de l'invention.

### EXEMPLES

### Exemple de référence 1 : chlorhydrate de 6-amino-N-[3,5-bis-(1,1-diméthyléthyl)-4-hydroxyphényl]-4-méthyl-2-pyridinebutanamide : R1

### 1.1) 6-(2,5-diméthylpyrrol-1-yl)-4-méthyl-2-{4-[(3,4,5,6-tétrahydro-2H-pyran-2-yl) oxy]butyl}-pyridine (R1.1) :

Dans un tricol, sous atmosphère d'argon, on dissout 2 g (10 mmoles) de 2-(2,5-diméthyl-pyrrol-1-yl)-4,6-diméthyl-pyridine dans 15 ml d'éther éthylique anhydre auxquels on ajoute une quantité catalytique de NaI. L'ensemble est refroidi à - 25° C avant l'addition goutte-à-goutte de 4,4 ml (11 mmoles) d'une solution 2,5 M de BuLi dans l'hexane, suivis 15 minutes plus tard de 1,65 ml (10 mmoles) de 2-(3-chloropropoxy)tétrahydro-2H-pyranne. Après 10 minutes d'agitation à -25° C, on laisse revenir lentement la température du mélange réactionnel à 23° C pendant une nuit. L'ensemble est finalement dilué par 20 ml d'une solution saturée de chlorure d'ammonium suivi de 30 ml d'acétate d'éthyle. Après décantation, la phase aqueuse est réextraite par 20 ml d'acétate d'éthyle, les phases organiques sont ensuite rassemblées et lavées successivement par 30 ml d'eau et 20 ml de saumure. La solution organique est séchée sur sulfate de sodium, filtrée et concentrée sous vide. Le résidu d'évaporation est purifié sur une colonne de silice (éluant : heptane/acétate d'éthyle : 9/1). On obtient le produit attendu sous forme d'une huile jaune pâle avec un rendement de 82 %.

RMN ¹H (CDCl₃, 400 MHz, δ) : 1,51-1,85 (m, 10H, 5 CH₂) ; 2,11 (s, 6H, 2 CH₃ pyrrole) ; 2,38 (s, 3H, CH₃) ; 2,80 (t, 2H, CH₂, J = 7,64 Hz) ; 3,46 (m, 2H, CH₂) ; 3,80 (m, 2H, CH₂) ; 4,57 (m, 1H, CH-O) ; 5,87 (s, 2H, pyrrole) ; 6,84 (s, 1H, pyridine) ; 6,97 (s, 1H, pyridine).

### 1.2) 6-(2,5-diméthylpyrrol-1-yl)-4-méthyl-2-pyridinebutanol (R1.2):

On dissout 2,08 g (6,07 mmoles) de l'intermédiaire R1.1 dans 10 ml (12,1 mmoles) d'une solution à 5 % d'HCl dans le méthanol. Après 2 heures d'agitation à 23° C, le mélange réactionnel est neutralisé par addition d'une solution saturée de NaHCO₃, concentré partiellement sous vide et finalement dilué par 30 ml d'acétate d'éthyle. La phase organique est décantée, lavée par 20 ml d'eau suivi de 10 ml de saumure, séchée sur sulfate de sodium, filtrée et concentrée sous vide. Le résidu d'évaporation est purifié sur une colonne de silice (éluant : heptane/acétate d'éthyle : 6/4). Les fractions pures sont collectées et évaporées pour conduire à une huile incolore avec un rendement de 77 %.

RMN ¹H (CDCl₃, 400 MHz, δ) : 1,60 (m, 2H, CH₂) ; 1,83 (m, 2H, CH₂) ; 2,11 (s, 6H, 2 CH₃ pyrrole) ; 2,39 (s, 3H, CH₃) ; 2,81 (t, 2H, CH2, J = 7,63 Hz) ; 3,64 (t, 2H, CH₂, J = 6,38 Hz) ; 5,88 (s, 2H, pyrrole) ; 6,86 (s, 1H, pyridine) ; 6,99 (s, 1H, pyridine).

### 1.3) 6-(2,5-diméthylpyrrol-1-yl)-4-méthyl-2-pyridinebutanal (R1.3):

A une solution de 0,4 g (1,54 mmole) de l'intermédiaire R1.2 dans 5 ml de DMSO, on ajoute successivement 1,3 ml (9,2 mmoles) de triéthylamine et une solution de 0,74 g (4,6 mmoles) du complexe sulfurtrioxide-pyridine dans 5 ml de DMSO. Au bout d'une heure d'agitation à 30° C, le mélange réactionnel est versé dans 50 ml d'eau et le produit est extrait par 20 ml d'acétate d'éthyle. Après décantation, la phase organique est lavée par 20 ml d'eau suivi de 10 ml de saumure, séchée sur sulfate de sodium, filtrée et finalement concentrée à sec sous vide. Le résidu est purifié sur une colonne de silice (éluant : heptane/acétate d'éthyle : 8/2), les fractions pures sont collectées et évaporées sous vide pour conduire à une huile incolore avec un rendement de 70 %.

RMN ¹H (DMSO d6, 400 MHz, δ) : 2,40 (m, 2H, CH₂) ; 2,50 (s, 6H, 2 CH₃ pyrrole) ; 2,83 (s, 3H, CH₃) ; 2,93 (m, 2H, CH₃) ; 3,18 (t, 2H, CH₃, J = 7,36 Hz) ; 6,23 (s, 2H, pyrrole) ; 7,52 (s, 1H, pyridine) ; 7,60 (s, 1H, pyridine) ; 10,12 (s, 1H, CHO).

### 1.4) acide 6-(2,5-diméthylpyrrol-1-yl)-4-méthyl-2-pyridinebutanoïque (R1.4):

A une solution de 0,61 g (2,38 mmoles) de l'intermédiaire R1.3 dans 20 ml éthanol, on ajoute successivement une solution de 1,02 g (5,95 mmoles) de AgNO₃ dans 4 ml d'eau et goutte-à-goutte une solution de 1,73 g (31 mmoles) de KOH dans 30 ml d'eau. Un précipité noir se forme rapidement et l'ensemble est agité pendant 3 heures à 22° C. Le mélange réactionnel est filtré et le filtrat est acidifié par une solution molaire d'HCl jusqu'à pH = 3. La solution aqueuse est extraite par 3 fois 20 ml de dichlorométhane, les phases organiques sont collectées, filtrées sur papier et le filtrat est lavé par 2 fois 20 ml de saumure. La solution organique est séchée sur sulfate de sodium, filtrée et concentrée sous vide. On obtient une huile jaune foncée avec un rendement de 92 %. Le produit est utilisé tel quel dans la réaction suivante.

### 1.5) N-[3,5-bis-(1,1-diméthyléthyl)-4-hydroxyphényl]-6-(2,5-diméthylpyrrol-1-yl)-4-méthyl-2-pyridinebutanamide (R1.5):

Le protocole expérimental utilisé est le même que celui décrit pour l'intermédiaire 1.5, l'intermédiaire R1.4 remplaçant l'intermédiaire 1.4. Le produit attendu est obtenu sous forme d'une poudre blanche avec un rendement de 41 %. Point de fusion = 78-80° C.

RMN ¹H (DMSO d6, 400 MHz, δ) : 1,34 (s, 18H, 2 tBu) ; 1,97 (m, 2H, CH₂) ; 2,04 (s, 6H, 2 CH₃ pyrrole) ; 2,27 (m, 2H, CH₂) ; 2,35 (s, 3H, CH₃) ; 2,73 (m, 2H, CH₂) ; 5,76 (s, 2H, pyrrole) ; 6,71 (s, 1H, OH) ; 7,04 (s, 1H, pyridine) ; 7,13 (s, 1H, pyridine) ; 7,38 (s, 2H, Ph) ; 9,56 (s, 1H, CO-NH).

### 1.6) chlorhydrate de 6-amino-N-[3,5-bis-(1,1-diméthyléthyl)-4-hydroxyphényl]-4-méthyl-2-pyridinebutanamide (R1.6):

Le protocole expérimental utilisé est le même que celui décrit pour l'intermédiaire 1.6, l'intermédiaire R1.5 remplaçant l'intermédiaire 1.5. Après salification de la base libre dans des conditions précédemment décrites, on obtient le chlorhydrate sous forme d'une poudre mauve avec un rendement de 56%. Point de fusion : 164-166° C.

RMN ¹H (DMSO d6, 400 MHz, δ) : 1,34 (s, 18H, 2 tBu), 1,96 (m, 2H, CH₂), 2,24 (s, 3H, CH₃), 2,28 (m, 2H, CH₂), 2,68 (m, 2H, CH₂), 6,57 (s, 1H, pyridine), 6,60 (s, 1H, pyridine), 6,75 (s, 1H, OH), 7,38 (s, 2H, Ph), 7,80 (s large, 2H, NH₂), 9,69 (s, 1H, CO-NH), 13,93 (s large, 1H, NH+).
IR : ν_{C=O} (amide) : 1662 cm⁻¹.

### Exemple de référence 2: chlorhydrate de N-[(6-amino-4-méthyl-2-pyridinyl) butyl]-2-hydroxy-5-methoxy-benzamide : R2

### 2.1) 6-(2,5-diméthyl-1H-pyrrol-1-yl)-4-méthyl-2-pyridinebutanamine (R2.1) :

Le protocole expérimental utilisé est le même que celui décrit pour l'intermédiaire 1.1, le 1-(3-bromopropyl)-2,2,5,5-tétraméthyl-1-aza-2,5-disilacyclopentane remplaçant le 4-bromobutyrate de triméthyle. On obtient une huile jaune avec un rendement de 62 %.

RMN ¹H (CDCl₃, 400 MHz, δ) : 1,52 (m, 2H, CH₂) ; 1,78 (m, 2H, CH₂) ; 2,11 (s, 6H, 2 x CH₃ pyrrole) ; 2,39 (s, 3H, CH₃ pyridine) ; 2,72 (t, 2H, CH₂, J = 7,02 Hz) ; 2,79 (t, 2H, CH₂, J = 7,62 Hz) ; 5,87 (s, 2H, pyrrole) ; 6,85 (s, 1H, pyridine) ; 6,98 (s, 1H, pyridine).

### 2.2) N-{4[6-(2,5-diméthyl-1H-pyrroi-1-yl)-4-méthyl-2-pyridinyl]butyl}-2-hydroxy-5-méthoxy benzamide (R2.2):

A une solution de 0,336 g (2 mmoles) d'acide 2-hydroxy-5-méthoxy-benzoïque dans 20 ml de dichlorométhane, on ajoute successivement 0,515 g (2 mmoles) d'intermédiaire R2.1, 0,3 ml de triéthylamine, 0,27 g (2 mmoles) d'hydroxybenzotriazole et 0,383 g (2 mmoles) de chlorhydrate de 1-(3-diméthylaminopropyl)-3-éthyl-carbodiimide. Après avoir agité le mélange réactionnel une nuit à 25° C, on dilue l'ensemble avec 40 ml d'eau et l'agitation est maintenue 10 minutes supplémentaires. Le produit est finalement extrait à l'aide de 2 fois 50 ml de dichlorométhane. La solution organique est séchée sur sulfate de sodium, filtrée et concentrée sous vide. Le résidu d'évaporation est purifié sur une colonne de silice (éluant acétate d'éthyle/heptane : 7/3) pour conduire à une huile jaune avec un rendement de 47 %.

RMN ¹H (DMSO d6, 400 MHz, δ) : 1,56 (m, 2H, CH₂) ; 1,71 (m, 2H, CH₂) ; 2,00 (s, 6H, 2 x CH₃ pyrrole) ; 2,34 (s, 3H, CH₃ pyridine) ; 2,73 (m, 2H, CH₂) ; 3,30 (m, 2H, CH₂) ; 3,71 (s, 3H, OCH₃) ; 5,75 (s, 2H, pyrrole) ; 6,80-7,38 (m, 5H, arom.) ; 8,83 (t large, 1H, CONH, J = 5,44 Hz) ; 12,20 (s large, 1H, OH arom.).

### 2.3) chlorhydrate de N-[(6-amino-4-methyl-2-pyridinyl)butyl]-2-hydroxy-5-methoxybenzamide (R2.3):

Le protocole expérimental utilisé est le même que celui décrit pour le composé 1.6, l'intermédiaire R2.2 remplaçant l'intermédiaire 1.5. On obtient un solide couleur mauve avec un rendement de 29 %. Point de fusion : 131-134° C.

RMN ¹H (DMSO d6, 400 MHz, δ) : 1,55 (m, 2H, CH₂) ; 1,69 (m, 2H, CH₂) ; 2,27 (s, 3H, CH₃ pyridine) ; 2,69 (t, 2H, CH₂, J = 7,4 Hz) ; 3,31 (m, 2H, CH₂) ; 3,73 (s, 3H, OCH₃) ; 6,59-7,45 (m, 5H, arom.) ; 7,74 (s large, 2H, NH₂) ; 8,96 (t, 1H, CONH, J = 5,36 Hz), 12,20 (s large, 1H, NH⁺) ; 13,93 (s large, 1H, OH arom.).
IR : ν_{C=O} (amide) : 1640-1660 cm⁻¹.

### Exemple de référence 3 : chlorhydrate de 6-amino-N-[3,5-bis(1,1-diméthyléthyl)-4-hydroxyphényl]-4-méthyl-2-pyridineheptanamide : R3

### 3.1) 6-(2,5-diméthyl-1H-pyrrol-1-yl)-4-méthyl-2-pyridine-heptanol (R3.1):

Le protocole expérimental utilisé est le même que celui décrit pour l'intermédiaire R1.1, le dérivé triméthylsilylé du 6-bromo-1-hexanol *(J. Org. Chem.,* (1988), **53** (12), 2732-7) remplaçant le 2-(3-chloropropoxy)tétrahydro-2H-pyranne. Le produit brut obtenu est ensuite dissous dans du THF et traité par une solution (1M dans le THF) de fluorure de tétrabutylammonium (1,2-1,5 éq.) à 20° C. Après une heure d'agitation, on ajoute goutte à goutte une solution saturée de chlorure d'ammonium. Le mélange est finalement concentré sous vide et le résidu repris par du dichlorométhane. La phase organique est lavée par de l'eau suivi de saumure, séchée sur sulfate de sodium, filtrée et concentrée sous vide. Le résidu est purifié sur une colonne de gel de silice (éluant dichlorométhane/méthanol : 98/2). On obtient une huile jaune clair avec un rendement de 79 %.

### 3.2) chlorhydrate de 6-amino-N-[3,5-bis(1,1-diméthyléthyl)-4-hydroxyphényl]-4-méthyl-2-pyridineheptanamide (R3.2):

Le protocole expérimental utilisé est le même que celui décrit pour les intermédiaires successifs R1.3 à R1.6, à partir du dérivé alcoolique R3.1 au lieu de l'intermédiaire R1.2. Poudre blanche. Point de fusion : 149-151° C.

RMN ¹H (DMSO d6, 400 MHz, δ) : 1,20-1,45 (m, 22H, 2 x tBu + 2 x CH₂) ; 1,56 (s large, 2H, CH₂) ; 1,64 (s large, 2H, CH₂) ; 2,23 (t, 2H, CH₂) ; 2,27 (s, 3H, CH₃ pyridine) ; 2,63 (t, 2H, CH₂) ; 6,57 (s, 1H, pyridine) ; 6,59 (s, 1H) ; 7,39 (s, 2H, arom.) ; 7,72 (s large, 2H, NH₂) ; 9,59 (s, 1H) ; 13,78 (s, 1H, NH⁺).
IR : ν_{C=O} (amide) : 1656 cm⁻¹.

### Exemple de référence 4 : chlorhydrate de 6-amino-N-[3,5-bis(1,1-diméthyléthyl)-4-hydroxyphényl]-4-méthyl-2-pyridinehexanamide : R4

Le protocole expérimental utilisé est le même que celui décrit pour le composé de référence R3, le dérivé triméthylsilylé du 5-chloro-1-pentanol (préparé selon *J. Org. Chem*., (1988), **53** (12), 2732-7) remplaçant le dérivé triméthylsilyl du 6-bromo-1-hexanol. Solide blanc cassé. Point de fusion : 101-103° C.

RMN ¹H (DMSO d6, 400 MHz, δ) : 1,34 (m, 20H, 2 x tBu + CH₂) ; 1,57 (m, 2H, CH₂) ; 1,65 (m, 2H, CH₂) ; 2,25 (m, 5H, CH₂ + CH₃ pyridine) ; 2,65 (t, 2H, CH₂) ; 6,59 (s, 2H, pyridine + OH) ; 6,72 (s, 1H, pyridine) ; 7,39 (s, 2H, arom.) ; 7,74 (s large, 2H, NH₂) ; 9,61 (s, 1H, CO-NH) ; 13,82 (s, 1H, NH⁺).
IR: ν_{C=O} (amide) : 1661 cm⁻¹.

### Exemple de référence 5 : N-[2-(benzyloxy)-4,5-diméthoxybenzyl]-4-[6-(2,5-diméthyl-1H-pyrrol-1-yl)-4-méthyl-2-pyridinyl]-1-butanamine : R5

Le protocole expérimental utilisé est le même que celui décrit pour l'intermédiaire 5.1, le 2-benzyloxy-4,5-diméthoxybenzaldéhyde remplaçant le 4-hydroxy-3-méthoxycinnamaldéhyde. Huile brune.

RMN ¹H (DMSO d6, 400 MHz, δ) : 1,45 (m, 2H, CH₂) ; 1,67 (m, 2H, CH₂) ; 2,01 (s, 6H, 2 x CH₃ (pyrrole)) ; 2,34 (s, 3H, CH₃) ; 2,50 (m, 2H, CH₂) ; 2,66 (m, 2H, CH₂) ; 3,64 (s, 2H, CH₂-NH) ; 3,66 (s, 3H, O-CH₃) ; 3,74 (s, 3H, O-CH₃) ; 4,40 (s large, 1H, NH) ; 5,07 (s, 2H, O-CH₂-Ph) ; 5,75 (s, 2H, pyridine) ; 6,70-7,50 (m, 9H, arom. + pyridine).

### Exemple de référence 6 : 6-(4-{[2-(benzyloxy)-4,5-diméthoxybenzyl]amino}butyl)-4-méthyl-2-pyridinamine : R6

Le protocole expérimental utilisé est le même que celui décrit pour l'intermédiaire 1.6, le composé de référence 5 remplaçant l'intermédiaire 1.5. On obtient la base libre sous forme d'une mousse brune.

RMN ¹H (CDCl₃, 400 MHz, δ) : 1,66 (m, 2H, CH₂) ; 1,80 (m, 2H, CH₂) ; 2,18 (s, 3H, CH₃) ; 2,43 (m, 2H, CH₂) ; 2,86 (m, 2H, CH₂) ; 3,82 (s, 3H, O-CH₃) ; 3,86 (s, 3H, O-CH₃) ; 4,14 (s, 2H, CH₂-NH) ; 5,05 (s, 2H, O-CH₂-Ph) ; 5,20-6,10 (m, 3H, NH₂ + NH) ; 6,20-7,40 (m, 9H, arom. + pyridine).
MS : MH+ = 436.

### Exemple 1 : chlorhydrate de 6-amino-N-[3,5-bis-(1,1-diméthyléthyl)-4-hydroxyphényl]-4-méthyl-2-pyridinepentanamide : 1

### 1.1) 6-(2,5-diméthylpyrrol-1-yl)-4-méthyl-2-pyridinepentanoate de méthyle :

Sous atmosphère d'argon, on dissout 0,4 g (2 mmoles) de 2-(2,5-diméthylpyrrol-1-yl)-4,6-diméthylpyridine (préparé à partir de la 6-amino-2,4-lutidine selon J. Chem. Soc. Perkin Trans. (1984), **12,** 2801-2807) dans 5 ml d'éther éthylique anhydre. Le mélange réactionnel est refroidi à -25° C et on ajoute goutte-à-goutte 0,9 ml (2,2 mmoles) d'une solution 2,5 M de BuLi dans l'hexane. Après 10 minutes à -25° C, on ajoute 0,35 ml (2 mmoles) de 4-bromobutyrate de triméthyle. Après avoir laissé la température remonter lentement à 23° C pendant la nuit, on ajoute 10 ml d'une solution saturée de chlorure d'ammonium au mélange réactionnel et on dilue finalement avec 10 ml d'acétate d'éthyle. La phase organique est décantée et lavée successivement par 10 ml d'eau et 10 ml de saumure, séchée sur sulfate de sodium filtrée et concentrée sous vide. Le résidu obtenu est purifié sur une colonne de silice (éluant : heptane/acétate d'éthyle : 95/5) pour conduire à une huile jaune avec un rendement de 55 %.

RMN ¹H (CDCl₃, 400 MHz, δ) : 1,70 (m, 2H, CH₂) ; 1,80 (m, 2H, CH₂) ; 2,10 (s, 6H, 2 CH₃ pyrrole) ; 2,35 (m, 5H, CH₂ + CH₃) ; 2,80 (m, 2H, CH₂) ; 3,65 (s, 3H, O-CH₃) ; 5,90 (s, 2H, pyrrole) ; 6,85 (s, 1H, pyridine) ; 6,95 (s, 1H, pyridine).

### 1.2) acide 6-(2,5-diméthylpyrrol-1-yl)-4-méthyl-2-pyridinepentanoïque :

On ajoute goutte-à-goutte une solution de 0,37 g (6,6 mmoles) de KOH dans 10 ml d'un mélange eau/méthanol (1/1) à une solution de 0,98 g (3,3 mmoles) de l'intermédiaire 1.1 dans 10 ml de méthanol. L'ensemble est agité 15 heures à 23° C et finalement dilué par 20 ml d'acétate d'éthyle. Après décantation, la phase aqueuse est lavée par 20 ml d'acétate d'éthyle et ensuite acidifiée à froid par une solution 2M d'acide chlorhydrique. Le produit est alors extrait par 2 fois 20 ml d'acétate d'éthyle. Après séchage sur sulfate de sodium et filtration, la solution organique est concentrée sous vide. Le résidu d'évaporation est purifié sur une colonne de silice (éluant : heptane/acétate d'éthyle : 7/3). Le produit attendu est obtenu sous forme d'une huile incolore avec un rendement de 64 %.

RMN ¹H (CDCl₃, 400 MHz, δ) : 1,70 (m, 2H, CH₂) ; 1,85 (m, 2H, CH₂) ; 2,10 (s, 6H, 2 CH₃ pyrrole) ; 2,40 (m, 5H, CH₂ + CH₃) ; 2,80 (m, 2H, CH₂) ; 5,90 (s, 2H, pyrrole) ; 6,85 (s, 1H, pyridine) ; 7,00 (s, 1H, pyridine).

### 1.3) 2,6-di-t-butyl-4-nitrophénol :

Le 2,6-di-t-butylphénol (8 g, 39 mmoles) est dissous dans 25 ml de cyclohexane à 10° C. Un mélange (1/1) acide nitrique / acide acétique (5 ml) est ajouté goutte à goutte dans le milieu réactionnel maintenu à cette température. On agite ensuite pendant 15 minutes à température ambiante, puis on filtre le précipité formé, on le rince par de l'eau et du pentane. Le 2,6-di-t-butyl-4-nitrophénol obtenu (6,34 g, 65 %) est séché à l'étuve et sera utilisé sans autre purification dans les étapes suivantes. Poudre jaune pâle. Point de fusion : 167-168° C.

RMN ¹H (CDCl₃, 100 MHz, δ) : 1,48 (s, 18H, 2tBu) ; 5,93 (s, 1H, OH) ; 8,13 (s, 2H, H arom.).

### 1.4) 2,6-di-t-butyl-4-aminophénol :

Le 2,6-di-t-butyl-4-nitrophénol (6,3 g, 25 mmoles) est dissous dans du méthanol (100 ml). On ajoute 0,6 g de palladium sur charbon (10 %) et on place l'ensemble sous atmosphère d'hydrogène sous 2 bars de pression. Le catalyseur est filtré et le solvant évaporé sous pression réduite. Le résidu est repris dans l'heptane et filtré. On obtient ainsi le 2,6-di-t-butyl-4-aminophénol (2,7 g, 48 %) qui sera utilisé sans autre purification dans les étapes suivantes. Poudre rose. Point de fusion : 123-124° C.

RMN ¹H (CDCl₃, 100 MHz, δ) : 6,60 (s, 2H, Ph) ; 4,65 (s large, 1H, OH) ; 3,15 (s large, 2H, NH₂) ; 1,42 (s, 18H, 2x tBu).

### 1.5) N-[3,5-bis-(1,1-diméthyléthyl)-4-hydroxyphényl]-6-(2,5-diméthylpyrrol-1-yl)-4-méthyl-2-pyridinepentanamide :

A une solution de 0,59 g (2,06 mmoles) de l'intermédiaire 1.2, de 0,46 g (2,06 mmoles) de 2,6-di-t-butyl-4-aminophénol et de 0,31 g (2,27 mmoles) d'hydroxybenzotriazole dans 20 ml de dichlorométhane, on ajoute en une portion 0,47 g (2,27 mmoles) de 1,3-dicyclohexylcarbodiimide. Le mélange réactionnel est agité pendant une nuit, à 23° C, le précipité formé est filtré et le filtrat est concentré à sec sous vide. Le résidu est dissous dans 20 ml d'acétate d'éthyle et lavé successivement par 20 ml d'eau et 20 ml de saumure. La solution organique est séchée sur sulfate de sodium, filtrée, concentrée sous vide et le résidu d'évaporation est purifié sur colonne de silice (éluant : dichlorométhane/méthanol : 98/2). Les fractions pures sont collectées et évaporées sous vide pour conduire à une huile transparente avec un rendement de 39 %.

RMN ¹H (DMSO d6, 400 MHz, δ) : 1,35 (s, 18H, 2 tBu) ; 1,60 (m, 2H, CH₂) ; 1,70 (m, 2H, CH₂) ; 2,00 (s, 6H, 2 CH₃ pyrrole) ; 2,25 (m, 2H, CH₂) ; 2,35 (s, 3H, CH₃) ; 2,70 (m, 2H, CH₂) ; 5,75 (s, 2H, pyrrole) ; 6,70 (s, 1H, OH) ; 7,00 (s, 1H, pyridine) ; 7,10 (s, 1H, pyridine) ; 7,35 (s, 2H, Ph) ; 9,55 (s,1H, CO-NH).

### 1.6) chlorhydrate de 6-amino-N-[3,5-bis-(1,1-diméthyléthyl)-4-hydroxyphényl]-4-méthyl-2-pyridinepentanamide :

On dissout l'intermédiaire 1.5 (0,43 g, 0,88 mmole) dans 9 ml d'éthanol additionné de 3 ml d'eau et on ajoute 0,31 g (4,40 mmoles) de chlorhydrate d'hydroxylamine. Le mélange réactionnel est chauffé à reflux pendant 24 heures. Après retour à 22° C, l'ensemble est dilué par 10 ml d'une solution saturée de bicarbonate de sodium et le produit est extrait par 20 ml d'acétate d'éthyle. Après décantation, la solution organique est lavée successivement par 20 ml d'une solution saturée de bicarbonate de sodium et 10 ml de saumure, séchée sur sulfate de sodium, filtrée et concentrée sous vide. Le résidu d'évaporation est repris par de l'éther éthylique et filtré pour conduire à la base libre sous forme d'un solide beige avec un rendement de 76 %. Le produit est ensuite salifié par traitement de 0,27 g (0,67 mmole) de la base libre en solution dans 5 ml de méthanol sec avec 2,68 ml (2,68 mmoles) d'une solution anhydre d'HCl 1N dans l'éther éthylique. On obtient une poudre beige. Point de fusion : 162-164° C.

RMN ¹H (DMSO d6, 400 MHz, δ) : 1,35 (s, 18H, 2 tBu) ; 1,50-1,80 (m, 4H, 2 CH₂) ; 2,30 (s, 5H, CH₂ + CH₃) ; 2,70 (m, 2H, CH₂) ; 6,60 (s, 2H, pyridine) ; 6,70 (s, 1H, OH) ; 7,40 (s, 2H, Ph) ; 7,75 (s large, 2H, NH₂) ; 9,66 (s, 1H, CO-NH) ; 13,95 (s large, 1H, NH⁺).
IR : ν_{C=O} (amide) : 1657 cm⁻¹.

### Exemple 2 : N-[(6-amino-4-méthyl-2-pyridinyl)butyl]-2,5-dihydroxy-3-(1-méthyléthyl)-benzamide : 2

Le protocole expérimental utilisé est le même que celui décrit pour l'exemple de référence 2, l'acide 2,5-dihydroxy-3-isopropyl benzoïque (préparé selon la méthode décrite dans *Can. J. Chem*., (1972), **50**, 1276-82) remplaçant l'acide 2-hydroxy-5-méthoxy-benzoïque. On obtient un solide rose pâle avec un rendement de 14 %. Point de fusion: 164-165° C.

RMN ¹H (DMSO d6, 400 MHz, δ) : 1,13 (d, 6H, CH₃ (isopropyle), J = 6,5 Hz) ; 1,52-1,60 (m, 4H, 2 x CH₂) ; 2,08 (s, 3H, CH₃ pyridine) ; 2,44 (m, 2H, CH₂) ; 3,19 (m, 1H, CH) ; 3,26 (m, 2H, CH₂) ; 5,66 (s, 2H, NH₂) ; 6,05 (s, 1H, pyridine) ; 6,18 (s, 1H, pyridine) ; 6,82 (s, 1H, arom.) ; 7,04 (s, 1H, arom.) ; 8,74 (s large, 1H, CONH) ; 8,88 (s large, 1H, OH) ; 12,69 (s large, 1H, OH).

### Exemple 3 : chlorhydrate de 6-amino-N-[4-(diméthylamino)phényl]-4-méthyl-2-pyridinehexanamide : 3

Le protocole expérimental utilisé est le même que celui décrit pour le composé de référence 4, la N,N-diméthyl-p-phénylènediamine remplaçant le 2,6-di-t-butyl-4-aminophénol. Solide gris hygroscopique.

RMN ¹H (CD₃OD, 400 MHz, δ) : 1,43 (m, 2H, CH₂) ; 1,72 (m, 4H, 2 x CH₂) ; 2,18 (s, 3H, CH₃) ; 2,34 (t, 2H, CH₂) ; 2,55 (t, 2H, CH₂) ; 2,90 (s, 6H, 2 x CH₃) ; 6,25 (s, 1H, pyridine) ; 6,36 (s, 1H, pyridine) ; 6,77 (d, 2H, arom.) ; 7,35 (d, 2H, arom.).
MS : MH+ = 341,2.

### Exemple 4 : chlorhydrate de 6-amino-N-[4-(diméthylamino)phényl]-4-méthyl-2-pyridinepentananamide : 4

Le protocole expérimental utilisé est le même que celui décrit pour le composé **1**, la N,N-diméthyl-p-phénylènediamine remplaçant le 2,6-di-t-butyl-4-aminophénol. Solide gris hygroscopique.

RMN ¹H (CD₃OD, 400 MHz, δ) : 1,72 (m, 4H, 2 x CH₂) ; 2,18 (s, 3H, CH₃) ; 2,35 (t, 2H, CH₂) ; 2,56 (t, 2H, CH₂) ; 2,88 (s, 6H, 2 x CH₃) ; 6,24 (s, 1H, pyridine) ; 6,35 (s, 1H, pyridine) ; 6,76 (d, 2H, arom.) ; 7,34 (d, 2H, arom.).
MS : MH+ = 327,2.

### Exemple 5 : chlorhydrate de 6-amino-N-[3-(4-hydroxy-3-méthoxy-phényl)-2-propényl]-4-méthyl-2-pyridine-butanamine : 5

### 5.1) 6-(2,5-diméthyl-1H-pyrrol-1-yl)-N-[3-(4-hydroxy-3-methoxy-phényl)2-propényl]-4-méthyl-2-pyridine-butanamine :

A une solution de 0,39 g (1,5 mmole) de l'intermédiaire R2.1 dans 15 ml de méthanol, on ajoute 0,27 g (1,5 mmole) de 4-hydroxy-3-méthoxycinnamaldéhyde et 2 g de tamis moléculaire 3 Å activé. Le mélange réactionnel est agité 24 heures à 20° C avant l'addition, à 0 °C, de 0,06 g (1,65 mmole) de NaBH₄, l'agitation est maintenue, à 20° C, 24 heures supplémentaires. Finalement l'excès d'hydrure est détruit par addition de 5 ml d'eau et le mélange est filtré sur fritté. Le filtrat est concentré sous vide. Le résidu d'évaporation est purifié sur une colonne de silice (éluant : CH₂Cl₂/MeOH/NH₄OH à 20 % : 95/4,5/0,5). On obtient une huile brune avec un rendement de 40 %.

### 5.2) chlorhydrate de 6-amino-N-[3-(4-hydroxy-3-méthoxy-phényl)-2-propényl]-4-méthyl-2-pyridine-butanamine :

Le protocole expérimental utilisé est le même que celui décrit pour l'intermédiaire 1.6, l'intermédiaire 5.1 remplaçant l'intermédiaire 1.5. Solide brun. Point de fusion : 60-62 °C.

RMN ¹H (DMSO d6, 400 MHz, δ) : 1,65 (m, 2H, CH₂) ; 1,72 (m, 2H, CH₂) ; 2,28 (s, 3H, CH₃ pyridine) ; 2,69 (s large, 2H, CH₂) ; 2,94 (s large, 2H, CH₂) ; 3,66 (s large, 2H, CH₂) ; 3,72 (s, 3H, CH₃-O) ; 6,10 (m, 1H, -CH=CH-CH₂) ; 6,67 (d, 1H, -CH=CH-CH₂) ; 6,82 (d, 1H, arom.) ; 6,88 (d, 1H, arom.) ; 7,00 (s, 1H, arom.) ; 7,73 (s large, 2H, NH₂) ; 8,91 (s large, 2H, NH + OH) ; 9,23 (s, 1H, NH⁺).
MS : MH+ = 342,3.

### Exemple 6 : 2-({[4-(6-amino-4-méthyl-2-pyridinyl)butyl]amino}méthyl)-4,5-diméthoxyphénol : 6

Ce composé est obtenu par débenzylation du composé de référence R6 en présence d'hydrogène et de Pd/C dans des conditions classiques. Mousse beige.

RMN ¹H (DMSO d6, 400 MHz, δ) : 1,67 (m, 4H, 2 x CH₂) ; 2,22 (s, 3H, CH₃) ; 2,60 (m, 2H, CH₂) ; 2,86 (m, 2H, CH₂) ; 3,68 (s, 3H, O-CH₃) ; 3,70 (s, 3H, O-CH₃) ; 3,97 (s, 2H, CH₂-NH) ; 6,50-7,00 (m, 4H, arom. + pyridine) ; 7,21 (s large, 1H, OH) ; 9,10 (s large, 2H, NH₂) ; 14,00 (s large, 1H, NH).
MS : MH+ = 346.

### Etude pharmacologique des produits de l'invention

### Etude des effets sur la NO synthase constitutive neuronale de cervelet de rat

L'activité inhibitrice des produits de l'invention est déterminée par la mesure de leur effets sur la transformation par la NO synthase de la [³H]L-arginine en [³H]L-citrulline en accord avec la méthode modifiée de Bredt et Snyder (*Proc. Natl. Acad. Sci. USA,* (1990) **87**: 682-685). Des cervelets de rats Sprague-Dawley (300 g - Charles River) sont rapidement prélevés, disséqués à 4° C et homogénéisés dans un volume de tampon d'extraction (HEPES 50 mM, EDTA 1 mM, pH 7.4, pepstatin A 10 mg/ml, leupeptine 10 mg/ml). Les homogénats sont ensuite centrifugés à 21000 g pendant 15 min à 4° C. Le dosage se fait dans des tubes à essai en verre dans lesquels sont distribués 100 µl de tampon d'incubation contenant 100 mM d'HEPES (pH 7,4), 2 mM d'EDTA, 2,5 mM de CaCl₂, 2 mM de dithiotréitol, 2 mM de NADPH réduit et 10 µg/ml de calmoduline. On ajoute 25 µl d'une solution contenant 100 nM de [³H]L-arginine (Activité spécifique : 56.4 Ci/mmole, Amersham) et 40 µM de L-arginine non radioactive. La réaction est initiée en ajoutant 50 µl d'homogénat, le volume final étant de 200 µl (les 25 µl manquants sont soit de l'eau, soit le produit testé. Après 15 min, la réaction est stoppée avec 2 ml de tampon d'arrêt (20 mM d'HEPES, pH 5,5, 2 mM d'EDTA). Après passage des échantillons sur une colonne de 1 ml de résine DOWEX, la radioactivité est quantifiée par un spectromètre à scintillation liquide. Les composés des exemples 1, 13, 21, 24, 26 et 35 décrits ci-dessus présentent une CI₅₀ inférieure à 5 µM.

### Etude des effets sur la péroxidation lipidique du cortex cérébral de rat

L'activité inhibitrice des produits de l'invention est déterminée par la mesure de leurs effets sur le degré de péroxidation lipidique, déterminée par la concentration en malondialdéhyde (MDA). Le MDA produit par la péroxidation des acides gras insaturés est un bon indice de la péroxidation lipidique (H Esterbauer and KH Cheeseman, *Meth. Enzymol*. (1990) **186** : 407-421). Des rats mâles Sprague Dawley de 200 à 250 g (Charles River) ont été sacrifiés par décapitation. Le cortex cérébral est prélevé, puis homogénéisé au potter de Thomas dans du tampon Tris-HCl 20 mM, pH = 7,4. L'homogénat est centrifugé deux fois à 50000 g pendant 10 minutes à 4° C. Le culot est conservé à -80° C. Le jour de l'expérience, le culot est remis en suspension à la concentration de 1 g/ 15 ml et centrifugé à 515 g pendant 10 minutes à 4° C. Le surnageant est utilisé immédiatement pour la détermination de la péroxidation lipidique. L'homogénat de cortex cérébral de rat (500 µl) est incubé à 37° C pendant 15 minutes en présence des composés à tester ou du solvant (10 µl). La réaction de péroxidation lipidique est initiée par l'ajout de 50 µl de FeCl₂ à 1 mM, d'EDTA à 1 mM et d'acide ascorbique à 4 mM. Après 30 minutes d'incubation à 37° C la réaction est arrêtée par l'ajout de 50 µl d'une solution de di tertio butyl toluène hydroxylé (BHT, 0,2 %). Le MDA est quantifié à l'aide d'un test colorimétrique, en faisant réagir un réactif chromogène (R) le N-méthyl-2-phénylindole (650 µl) avec 200 µl de l'homogénat pendant 1 heure à 45° C. La condensation d'une molécule de MDA avec deux molécules de réactif R produit un chromophore stable dont la longueur d'onde d'absorbance maximale est égale à 586 nm. (Caldwell et coll. *European J. Pharmacol*. (1995) **285**, 203-206). Les composés des exemples 1 à 6 décrits ci-dessus présentent une CI₅₀ inférieure à 30 µM.

## Revendications

1. Composé choisi parmi les composés suivants :
- 6-amino-N-[3,5-bis-(1,1-diméthyléthyl)-4-hydroxyphényl]-4-méthyl-2-pyridinepentanamide ;
- N-[(6-amino-4-méthyl-2-pyridinyl)butyl]-2,5-dihydroxy-3-(1-méthyléthyl)-benzamide ;
- 6-amino-N-[4-(diméthylamino)phényl]-4-méthyl-2-pyridinehexanamide ;
- 6-amino-N-[4-(diméthylamino)phényl]-4-méthyl-2-pyridinepentananamide ;
- 6-amino-N-[3-(4-hydroxy-3-méthoxy-phényl)-2-propényl]-4-méthyl-2-pyridinebutanamine ;
- 2-({[4-(6-amino-4-méthyl-2-pyridinyl)butyl]amino}méthyl)-4,5-diméthoxyphénol ;
ou sel d'addition d'un de ces composés à des acides organiques ou inorganiques ou à des bases.

2. A titre de médicament, un composé tel que défini dans la revendication 1, ou un sel pharmaceutiquement acceptable d'un tel composé.

3. Composition pharmaceutique contenant, à titre de principe actif, au moins un composé tel que défini dans la revendication 1, ou un sel pharmaceutiquement acceptable d'un tel composé.

4. Utilisation d'un composé tel que défini dans la revendication 1, ou d'un sel pharmaceutiquement acceptable d'un tel composé pour fabriquer un médicament destiné à inhiber la NO synthase.

5. Utilisation d'un composé tel que défini dans la revendication 1, ou d'un sel pharmaceutiquement acceptable d'un tel composé pour fabriquer un médicament destiné à inhiber la peroxydation lipidique.

6. Utilisation d'un composé tel que défini dans la revendication 1, ou d'un sel pharmaceutiquement acceptable d'un tel composé, pour fabriquer un médicament ayant à la fois une activité d'inhibition de la NO synthase et d'inhibition de la peroxydation lipidique.

7. Utilisation d'un composé tel que défini dans la revendication 1, ou d'un sel pharmaceutiquement acceptable d'un tel composé, pour fabriquer un médicament destiné à traiter une pathologie choisie parmi les troubles cardio-vasculaires et cérébro-vasculaires, les troubles du système nerveux central ou périphérique, les troubles du muscle squelettique et des jonctions neuromusculaires, les maladies cutanées, les maladies prolifératives et inflammatoires, les maladies auto-immunes et virales et les maladies neurologiques associées à des intoxications, à des traitements ou à des désordres d'origine génétique.

8. Utilisation selon la revendication 7, **caractérisée en ce que** le médicament fabriqué est destiné à traiter des troubles cardio-vasculaires et cérébro-vasculaires.

9. Utilisation selon la revendication 8, **caractérisée en ce que** les troubles cardio-vasculaires et cérébro-vasculaires sont choisis parmi l'athérosclérose, la migraine, l'hypertension artérielle, le choc septique, les infarctus cardiaques ou cérébraux d'origine ischémique ou hémorragiques, les ischémies et les thromboses.

10. Utilisation selon la revendication 7, **caractérisée en ce que** le médicament fabriqué est destiné à traiter des troubles du système nerveux central ou périphérique.

11. Utilisation selon la revendication 10, **caractérisée en ce que** les troubles du système nerveux central ou périphérique sont choisis parmi les infarctus cérébraux, l'hémorragie sub arachnoïde, le vieillissement, les démences séniles, la maladie d'Alzheimer, la chorée de Huntington, la maladie de Parkinson, la maladie de Creutzfeld Jacob, les maladies à prions, la sclérose latérale amyotrophique, la douleur, les traumatismes cérébraux ou de la moelle épinière, l'addiction aux opiacées, à l'alcool et aux substances induisant une accoutumance, les troubles de l'érection et de la reproduction, les désordres cognitifs et les encéphalopathies.

12. Utilisation selon la revendication 7, **caractérisée en ce que** le médicament fabriqué est destiné à traiter la sclérose en plaques.

## Patentansprüche

1. Verbindung, die aus den folgenden Verbindungen ausgewählt ist:
- 6-Amino-N-[3,5-bis-(1,1-dimethylethyl)-4-hydroxyphenyl]-4-methyl-2-pyridinpentanamid;
- N-[(6-Amino-4-methyl-2-pyridinyl)butyl]-2,5-dihydroxy-3-(1-methylethyl)-benzamid;
- 6-Amino-N-[4-(dimethylamino)phenyl]-4-methyl-2-pyridinhexanamid;
- 6-Amino-N-[4-(dimethylamino)phenyl]-4-methyl-2-pyridinpentanamid;
- 6-Amino-N-[3-(4-hydroxy-3-methoxyphenyl)-2-propenyl]-4-methyl-2-pyridinbutanamin;
- 2-({[4-(6-Amino-4-methyl-2-pyridinyl)butyl]amino}methyl)-4,5-dimethoxyphenol;
oder ein Additionssalz einer dieser Verbindungen mit organischen oder anorganischen Säuren oder mit Basen.

2. Eine Verbindung, wie sie in Anspruch 1 definiert ist, oder ein pharmazeutisch annehmbares Salz einer solchen Verbindung in Form eines Medikaments.

3. Pharmazeutische Zusammensetzung, die als Wirkstoff mindestens eine Verbindung der in Anspruch 1 definierten Art oder ein pharmazeutisch annehmbares Salz einer solchen Verbindung enthält.

4. Verwendung einer Verbindung, wie sie in Anspruch 1 definiert ist, oder eines pharmazeutisch annehmbaren Salzes einer solchen Verbindung für die Herstellung eines Medikaments, das zur Hemmung der NO-Synthase bestimmt ist.

5. Verwendung einer Verbindung, wie sie in Anspruch 1 definiert ist, oder eines pharmazeutisch annehmbaren Salzes einer solchen Verbindung für die Herstellung eines Medikaments, das für die Hemmung der Lipidperoxidation bestimmt ist.

6. Verwendung einer Verbindung, wie sie in Anspruch 1 definiert ist, oder eines pharmazeutisch annehmbaren Salzes einer solchen Verbindung für die Herstellung eines Medikaments, das gleichzeitig eine die NO-Synthase hemmende Wirkung und eine die Lipidperoxidation hemmende Wirkung besitzt.

7. Verwendung einer Verbindung, wie sie in Anspruch 1 definiert ist, oder eines pharmazeutisch annehmbaren Salzes einer solchen Verbindung für die Herstellung eines Medikaments, das für die Behandlung einer Krankheit bestimmt ist, die aus den folgenden Krankheiten ausgewählt ist: kardiovaskuläre und zerebrovaskuläre Störungen, Störungen des zentralen oder peripheren Nervensystems, Störungen der Skelettmuskulatur und der neuromuskulären Verbindungen, Hautkrankheiten, proliferative und entzündliche Krankheiten, Autoimmun- und Viruserkrankungen und neurologische Erkrankungen in Zusammenhang mit Vergiftungen, mit Behandlungen oder mit Störungen genetischen Ursprungs.

8. Verwendung nach Anspruch 7, **dadurch gekennzeichnet, dass** das hergestellte Medikament für die Behandlung von kardiovaskulären und zerebrovaskulären Störungen bestimmt ist.

9. Verwendung nach Anspruch 8, **dadurch gekennzeichnet, dass** die kardiovaskulären und zerebrovaskulären Störungen aus den folgenden Störungen ausgewählt sind: Atherosklerose, Migräne, arterielle Hypertonie, septischer Schock, Herz- oder Hirninfarkte ischämischen oder hämorrhagischen Ursprungs, Ischämien und Thrombosen.

10. Verwendung nach Anspruch 7, **dadurch gekennzeichnet, dass** das hergestellte Medikament für die Behandlung von Störungen des zentralen oder peripheren Nervensystems bestimmt ist.

11. Verwendung nach Anspruch 10, **dadurch gekennzeichnet, dass** die Störungen des zentralen oder peripheren Nervensystems aus den folgenden Störungen ausgewählt sind: Hirninfarkte, subarachnoidale Hämorrhagie, Altern, Altersdemenzen, Alzheimer-Krankheit, Chorea-Huntington, Parkinson-Krankheit, Creutzfeld-Jacob-Krankheit, Prionenkrankheiten, amyotrophe Lateralsklerose, Schmerzen, Hirn- oder Rückenmarkverletzungen, Abhängigkeit von Opiaten, Alkohol und von Substanzen, die eine Gewöhnung erzeugen, Erektions- und Fortpflanzungsstörungen, kognitive Störungen und Enzephalopathien.

12. Verwendung nach Anspruch 7, **dadurch gekennzeichnet, dass** das hergestellte Medikament für die Behandlung der Plaques-Sklerose bestimmt ist.

## Claims

1. Compound chosen from the following compounds:
- 6-amino-N-[3,5-bis-(1,1-dimethylethyl)-4-hydroxyphenyl]-4-methyl-2-pyridinepentanamide;
- N-[(6-amino-4-methyl-2-pyridinyl)butyl]-2,5-dihydroxy-3-(1-methylethyl)-benzamide;
- 6-amino-N-[4-(dimethylamino)phenyl]-4-methyl-2-pyridinehexanamide;
- 6-amino-N-[4-(dimethylamino)phenyl]-4-methyl-2-pyridinepentananamide;
- 6-amino-N-[3-(4-hydroxy-3-methoxy-phenyl)-2-propenyl]-4-methyl-2-pyridinebutanamine;
- 2-({[4-6-amino-4-methyl-2-pyridinyl)butyl]amino}methyl)-4,5-dimethoxyphenol;
or an addition salt of one of these compounds with organic or inorganic acids or with bases.

2. As a medicament, a compound as defined in claim 1, or a pharmaceutically acceptable salt of such a compound.

3. Pharmaceutical composition containing, as active ingredient, at least one compound as defined in claim 1, or a pharmaceutically acceptable salt of such a compound.

4. Use of a compound as defined in claim 1, or a pharmaceutically acceptable salt of such a compound for producing a medicament intended to inhibit NO synthase.

5. Use of a compound as defined in claim 1, or a pharmaceutically acceptable salt of such a compound for producing a medicament intended to inhibit lipidic peroxidation.

6. Use of a compound as defined in claim 1, or a pharmaceutically acceptable salt of such a compound for producing a medicament having both an NO synthase inhibition activity and lipidic peroxidation inhibition activity.

7. Use of a compound as defined in claim 1, or a pharmaceutically acceptable salt of such a compound for producing a medicament intended to treat a pathology chosen from cardio-vascular and cerebro-vascular disorders, disorders of the central or peripheral nervous system, disorders of the skeletal muscle and neuromuscular joints, cutaneous diseases, proliferative and inflammatory diseases, auto-immune and viral diseases and neurological diseases associated with intoxications, treatments or disorders of genetic origin.

8. Use according to claim 7, **characterized in that** the medicament produced is intended to treat cardio-vascular and cerebro-vascular disorders.

9. Use according to claim 8, **characterized in that** the cardio-vascular and cerebro-vascular disorders are chosen from atherosclerosis, migraine, arterial hypertension, septic shock, ischemic or hemorragic cardiac or cerebral infarctions, ischemias and thromboses.

10. Use according to claim 7, **characterized in that** the medicament produced is intended to treat disorders of the central or peripheral nervous system.

11. Use according to claim 10, **characterized in that** the disorders of the central or peripheral nervous system are chosen from cerebral infarctions, sub-arachnoid haemorrhaging, ageing, senile dementias, Alzheimer's disease, Huntington's chorea, Parkinson's disease, Creutzfeld Jacob disease and prion diseases, amyotrophic lateral sclerosis, pain, cerebral and spinal cord traumas, addiction to opiates, alcohol and addictive substances, erective and reproductive disorders, cognitive disorders and encephalopathies.

12. Use according to claim 7, **characterized in that** the medicament produced is intended to treat multiple sclerosis.
